# EUROPEAN PATENT SPECIFICATION

(11) **EP 1 466 660 B1**
(45) Date of publication and mention of the grant of the patent: **24.12.2008**
(21) Application number: 03024972.6
(22) Date of filing: 29.10.2003
(51) Int. Cl.: B01D 69/14, C07C 7/144

(54) **Facilitated transport membranes**
Membranen zum erleichterten Transport
Membranes à transport facilité

(30) Priority: 11.04.2003 KR 2003022837
(43) Date of publication of application: 13.10.2004
(73) Proprietor: Korea Institute of Science and Technology, Seoul 136-791 (KR)
(72) Inventor: Kim, Jong Hak, Dobong-gu Seoul 132-741 (KR); Kang, Yong Soo, Seongbuk-gu Seoul 136-784 (KR); Jung, Bumsuk, Seongbuk-gu Seoul 136-791 (KR); Won, Jongok, Dongdaemun-gu Seoul 130-775 (KR); Min, Byoung Ryul, Seodaemun-gu Seoul 120-160 (KR); Kim, Hoon Sik, Seongbuk-gu Seoul 136-767 (KR)
(74) Representative: Diehl & Partner

(56) References cited:
- EP-A- 0 634 204
- DE-A- 19 929 482
- US-A- 5 062 866
- US-A- 5 378 440

## Description

The present invention relates to a facilitated transport membrane with an improved permeance and selectivity to alkene hydrocarbons. In particular, the present invention relates to a facilitated transport membrane prepared by forming a solid polymer electrolyte consisting of a transition metal salt and a polymer having double carbon bonds capable of forming a π -complex with an ion of the transition metal salt, and coating the solid polymer electrolyte on a porous supported membrane with good permeance and superior mechanical strength. The facilitated transport membrane is characterized in that its permeance and selectivity to alkene hydrocarbons is high and in that the complex of a metal and a polymer ligand in the solid polymer electrolyte maintains its activity as a carrier for alkene hydrocarbons even under long-term dry operating conditions.

Alkene hydrocarbons are primarily produced by pyrolysis of naphtha obtained from a petroleum refining process. They are important raw materials that form the basis of the current petrochemical industry. However, they are generally produced along with alkane hydrocarbons such as ethane and propane. Thus, alkene hydrocarbons/alkane hydrocarbons separation technology is of significant importance in the related industry.

Currently, the traditional distillation process is used mostly for the separation of alkene/alkane mixtures such as ethylene/ethane or propylene/propane. The separation of such mixtures, however, requires the investment of large-scale equipment and high-energy cost due to their similarity in molecular size and physical properties such as relative volatility.

In the distillation process used hitherto, for example, a distillation column having about 120-160 trays should be operated at a temperature of -30°C and a high pressure of about 20 atm for separation of an ethylene and ethane mixture. For separation of a propylene and propane mixture, a distillation column having about 180-200 trays should be operated at a temperature of -30°C and a pressure of about several atms in the reflux ratio of 10 or more. As such, there has been a continuous need for the development of a new separation process that can replace the prior distillation process, which requires the investment of large-scale equipment and high-energy cost.

A separation process that could be considered as a replacement for said prior distillation process is one that uses a separation membrane. Separation membrane technology has progressed remarkably over the past few decades in the field of separating gas mixtures, for example, the separation of nitrogen/oxygen, nitrogen/carbon dioxide and nitrogen/methane, etc.

However, the satisfactory separation of alkene/alkane mixtures cannot be accomplished by using traditional gas separation membranes because alkene and alkane are very similar in terms of their molecular size and physical properties. A facilitated transport membrane based on a different concept from the traditional gas separation membranes is considered to be a separation membrane that can achieve excellent separation performance for alkene/alkane mixtures.

The separation of mixtures in a separation process using a separation membrane is achieved by the difference in permeance between the individual components constituting the mixtures. Most materials of a separation membrane have many limitations on their application because of an inverse correlation between permeance and selectivity. However, the concurrent increase of permeance and selectivity is made possible by applying a facilitated transport phenomenon. Consequently, the scope of their application can be considerably increased. If a carrier capable of selectively and reversibly reacting with a specific component of a mixture is present in a separation membrane, mass transport is facilitated by additional material transport resulting from a reversible reaction of a carrier and a specific component. Therefore, overall mass transport can be indicated by Fick's law and the sum of material transport caused by a carrier. This phenomenon is referred to as facilitated transport.

A supported liquid membrane is an example of a membrane prepared by applying the concept of facilitated transport. The supported liquid membrane is prepared by filling a porous thin layer with solution that is obtained by dissolving a carrier capable of facilitating mass transport in a solvent such as water, etc. Such a supported liquid membrane has succeeded to a certain extent.

Steigelmann and Hughes, for example, prepare a supported liquid membrane in which the selectivity of ethylene/ethane is about 400-700 and the permeance of ethylene is 60 GPU [1 GPU = 1 x 10⁻⁶ cm³ (STP)/cm²· sec· cmHg], which are satisfactory performance results for permeance separation (see U.S. Pat. Nos. 3,758,603 and 3,758,605). However, the supported liquid membrane exhibits the facilitated transfer phenomenon only under wet conditions. There is the inherent problem that the initial permeance separation performance cannot be maintained for an extended time due to solvent loss and the decrease of separation performance over time.

In order to solve the problem, Kimura, etc., suggests a method that enables facilitated transport by substituting a suitable ion in an ion-exchange resin (see U.S. Pat. No. 4,318,714). This ion-exchange resin membrane also has a drawback, however, in that the facilitated transport phenomenon is exhibited only under wet conditions, similar to the supported liquid membrane.

According to the patent document US 5,062,866 to Ho, a membrane is provided for separating aliphatically unsaturated hydrocarbons from hydrocarbon mixtures in a liquid water environment, the membrane comprising a hydrophilic polymer which contains metals capable of complexing with the hydrocarbons, and a hydrophilic salt of a Group I metal.

Ho suggests another method for the preparation of a complex by using watersoluble glassy polymer such as polyvinyl alcohol (see U.S. Pat. Nos. 5,015,268 and 5,062,866). However, the method also has a drawback in that satisfactory results are obtained only when feed gas is saturated with water vapor by passing the feed gas through water or when a membrane is swelled with ethylene glycol or water.

In all the instances described above, the separation membrane must to be maintained in wet conditions to contain water or other similar solvents. When a dry hydrocarbon gas mixture - for example, an alkene/alkane mixture free of a solvent such as water - is separated by using the membrane, solvent loss is unavoidable with time. Therefore, a method is necessary for periodically feeding a solvent to a separation membrane in order to continuously sustain the wet condition of the separation membrane. It is, however, rarely possible for the method to be applied to a practical process, and the membrane is not stable.

Kraus, etc., develops a facilitated transport membrane by using another method (see U.S. Pat. No. 4,614,524). According to the patent, a transition metal is substituted in an ion-exchange membrane such as Nafion, and the membrane is plasticized with glycerol, etc. The membrane could not be utilized, however, in that its selectivity is as low as about 10 when dry feed is used. The membrane also has no selectivity when a plasticizer is not used. Furthermore, a plasticizer is lost with time.

In view that a usual polymer separation membrane cannot separate alkene/alkane mixtures having similar molecular size and physical properties, as described above, use of a facilitated transport membrane capable of selectively separating only alkane is necessary. In conventional facilitated transport membranes, however, the activity of a carrier is maintained by using the following method: filling a solution containing a carrier into the porous membrane, adding a volatile plasticizer, or saturating a feed gas with water vapor. Such a membrane cannot be utilized due to the problem of declining stability of the membrane since components constituting the membrane are lost with time. There is also the problem of later having to remove solvents such as water, etc., which are periodically added in order to sustain activity, from the separated product.

Therefore, there is a need for the development of a separate membrane that can replace the prior distillation process requiring the investment of large-scale equipment and high-energy cost in the separation of alkene/alkane mixtures, in which the separation membrane does not contain volatile components and has high selectivity and permeance so that it can maintain the activity even under long-term dry operating conditions.

The purpose of the present invention is to prepare a facilitated transport membrane by introducing the principle of a non-volatile polymer electrolyte used in a polymer battery into said facilitated transport membrane, in which the membrane has a high permeance and selectivity to unsaturated hydrocarbons such as alkene even under dry conditions and has no problems in stability, such as carrier loss, to be able to sustain the activity for a prolonged period of time.

That is, an object of the present invention is to prepare a facilitated transport membrane having its prominent characteristics in separating alkene hydrocarbons from mixtures of alkene hydrocarbons and alkane hydrocarbons by coating a solid polymer electrolyte consisting of a transition metal salt and a polymer having double carbon bonds on a porous supported membrane. The facilitated transport membrane prepared according to the present invention has a high permeance and selectivity to alkene and maintains the activity even under long-term dry operating conditions with no feed of liquid solvents.

In the facilitated transport membrane prepared according to the present invention, a polymer ligand and a metal ion of a transition metal salt in a non-volatile polymer electrolyte form a complex. The metal ion of the complex then reacts selectively and reversibly with a double bond of alkene, resulting in the facilitated transport of alkene. Thus, the membrane can selectively separate alkene hydrocarbon.

A transition metal-polymer electrolyte prepared by using a polymer having double carbon bonds does not exhibit the performance deterioration of a transition metal-polymer electrolyte prepared by a polymer having a functional group including oxygen and/or nitrogen, and especially does not exhibit the reduction of a transition metal ion to a transition metal. Thus, the electrolyte prepared by using a polymer having double carbon bonds has good resistance to heat and to ultraviolet and visible lights.

The present invention is described in detail below.

The facilitated transport membrane according to the present invention comprises a solid polymer electrolyte, consisting of a transition metal salt and a polymer having double carbon bonds and having a selective permeance to alkene hydrocarbon, and a porous supported membrane supporting it.

Hydrocarbon mixtures to be separated in the present invention contain at least one alkene hydrocarbon and at least one alkane hydrocarbon or inert gas. The alkene hydrocarbon includes ethylene, propylene, butylene, 1,3-butadiene, isobutylene, isoprene, etc.; the alkane-type hydrocarbon includes methane, ethane, propane, butane, isobutane, etc.; and the inert gas includes oxygen, nitrogen, carbon dioxide, carbon monoxide, water, etc.

Any porous supported membranes having good permeance and sufficient mechanical strength may be used in the present invention. For example, both a conventional porous polymer membrane and a ceramic membrane may be used. Plate, tubular, hollow or other types of supported membranes may also be used in the invention.

A solid polymer electrolyte consists of a transition metal salt acting as a carrier and a non-volatile polymer having double carbon bonds. The transition metal salt in the electrolyte is not simply dispersed or mixed in the polymer. It is dissociated into a cation and an anion on the polymer because the ion of a transition metal interacts strongly with unsaturated hydrocarbon of the polymer to form a π -complex. Therefore, contrary to a conventional membrane, the separation membrane according to the present invention does not require the addition of water to sustain the activity of a carrier or the addition of other solvents to swell the polymer matrix. It also selectively facilitates the transport of a dry alkene hydrocarbon.

In the facilitated transport membrane according to the present invention, the electrolyte consisting of a transition metal salt acting as a carrier and a polymer having double carbon bonds has a substantial effect on the selective separation of alkene hydrocarbon. The properties of the electrolyte determine the selective permeation separation of alkene hydrocarbon from the corresponding alkane hydrocarbon.

The transition metal salt consists of a cation of a transition metal and an anion of a salt, and it is dissociated into ions on the polymer. The cation reacts reversibly with a double bond of alkene hydrocarbon to form a complex and directly participate in the facilitated transport. That is, a cation of a transition metal in the electrolyte interacts with an anion of salt, a polymer and alkene hydrocarbon. Therefore, they must be properly selected to obtain a separation membrane having high selectivity and permeance. The stability of both the selected polymer and the formed metal complex also serves an important role in long-term operation.

It is well known that a transition metal reacts reversibly with alkene hydrocarbon in a solution (see J. P. C. M. Van Dongen, C. D. M. Beverwijk, J. Organometallic Chem. 1973, 51, C36). The ability of a transition metal ion as a carrier is determined by the size of the π -complexation formed with alkene, which is determined by electronegativity. Electronegativity is a measure of the relative strength of an atom to attract covalent electrons when the atom is bonded with other atoms. The electronegativity values of transition metals are shown in Table 1 below.

**Table 1**

| Electronegativity Values of Transition Metals | | | | | | |
|---|---|---|---|---|---|---|
| Transition metal | Sc | V | Cr | Fe | Ni | Cu |
| Electronegativity | 1.4 | 1.6 | 1.7 | 1.8 | 1.9 | 1.9 |
| Transition metal | Y | Nb | Mo | Ru | Pd | Ag |
| Electronegativity | 1.3 | 1.6 | 2.2 | 2.2 | 2.2 | 1.9 |
| Transition metal | La | Ta | W | Os | Pt | Au |
| Electronegativity | 1.0 | 1.5 | 2.4 | 2.2 | 2.3 | 2.5 |

If the electronegativity of a metal is high, the metal atom will more strongly attract electrons when it is bonded with other atoms. If the electronegativity of a metal is too high, the metal is not suitable as a carrier of the facilitated transport due to increased possibility of the irreversible reaction of the metal and π -electrons of alkene. On the other hand, if the electronegativity of a metal is too low, the metal cannot act as a carrier because of its low interaction with alkene.

Therefore, the electronegativity of a metal is preferably in the range of from 1.6 to 2.3 so that the transition metal ion reacts reversibly with alkene. Preferred transition metals within the above ranges include Mn, Fe, Co, Ni, Cu, Mo, Tc, Ru, Rh, Pd, Ag, Re, Os, Ir, Pt, or complexes thereof, etc.

An anion of a transition metal has an important role in improving the reversible reactivity of a metal transition ion and alkene hydrocarbons, particularly in improving the reverse reaction rate, allowing readily separation of alkenes that form a complex with a transition metal in effluent. For a transition metal to act as a carrier of alkenes, a transition metal salt MX should be solvated on a polymer and form a complex as shown in Scheme 1 below.

MX+[G] ⇒ M-X[G] [Scheme 1]

Wherein [G] and M-X-[G] represent a functional group of a polymer and a complex, respectively. The difference in the solvation tendency of an anion on a polymer is generally dependent on the difference in dielectric constant of the polymer. If the polarity of the polymer is low, however, the solvation stability of most anions is generally reduced. The lower the lattice energy of a transition metal salt, the lesser the tendency of an anion and cation to form strong ion pairs. As a result, the decrease in solvation stability of an anion is relieved.

Therefore, it is preferable to select an anion of a transition metal salt that has low lattice energy in respect of a given cation of a transition metal, in order to readily solvate a transition metal salt and improve solvation stability in the facilitated transport membrane according to the present invention. The lattice energy of representative transition metal salts is given in Table 2 below.

**Table 2**

| Lattice Energy of Metal Salts [kJ/mol]^{a)} | | | | | | | | | | |
|---|---|---|---|---|---|---|---|---|---|---|
| | Li⁺ | Na⁺ | K⁺ | Ag⁺ | Cu⁺ | Co²⁺ | Mo²⁺ | Pd²⁺ | Ni²⁺ | Ru³⁺ |
| F⁻ | 1036 | 923 | 823 | 967 | 1060^{b)} | 3018 | | | 3066 | |
| Cl⁻ | 853 | 786 | 715 | 915 | 996 | 2691 | 2733 | 2778 | 2772 | 5245 |
| Br⁻ | 807 | 747 | 682 | 904 | 979 | 2629 | 2742 | 2741 | 2709 | 5223 |
| I⁻ | 757 | 704 | 649 | 889 | 966 | 2545 | 2630 | 2748 | 2623 | 5222 |
| CN⁻ | 849 | 739 | 669 | 914 | 1035 | | | | | |
| NO₃⁻ | 848 | 756 | 687 | 822 | 854^{b)} | 2626 | | | 2709 | |
| BF₄⁻ | 705 ^{b)} | 619 | 631 | 658 ^{b)} | 695 ^{b)} | 2127 | | | 2136 | |
| ClO₄⁻ | 723 | 648 | 602 | 667^{b)} | 712 ^{b)} | | | | | |
| CF₃SO₃⁻ | 779^{b)} | 685^{b)} | 600^{b)} | 719^{b)} | 793^{b)} | | | | | |
| CF₃CO₂⁻ | 822 ^{b)} | 726^{b)} | 658^{b)} | 782^{b)} | 848^{b)} | | | | | |

| | | | | | | | | | | |
|---|---|---|---|---|---|---|---|---|---|---|
| ^{a)} See H.D.B. Jenkins, CRC Handbook, 74^{th} Ed., 12-13 (1993) ^{b)} Complexation energy for the formation of an ion pair such as M⁺_{(g)} + X⁻_{(g)} ⇒ MX_{(g)} is calculated by using the Becke3LYP method (Becke3/6-311 + G*//Becke3/6-311 + G*) of Density Function Theory (DFT), which uses a basic set function of 6-311+G*. The calculated value linear-regresses with the lattice energy described in literature a). It is confirmed that there is good linearity with a correlation coefficient of at least 0.94. Thus, the lattice energy of salts that are not described in the literature is estimated by using the correlation obtained above. | | | | | | | | | | |

An anion constituting a transition metal salt of the facilitated transport membrane according to the present invention is preferably selected from anions having a lattice energy of 2500 kJ/mol or less in order to suppress the tendency to form a strong ion pair with a cation and to improve solvation stability. Among the metal salts listed in Table 2, the anions may include F⁻, Cl⁻, Br⁻, I⁻, CN⁻, NO₃⁻ and BF₄⁻, which constitute salts with Ag⁺ or Cu⁺. Anions applicable to the present invention, however, are not limited only to those listed in Table 2.

The solution stability of anions is generally exhibited in the order of F⁻<< Cl⁻< Br⁻< I⁻∼ SCN⁻< ClO₄⁻∼ CF₃SO₃⁻< BF₄'∼ AsF₆⁻, in which lattice energy decreases, i.e., the tendency of the anions to form strong ion pairs with cations of metal salts is reduced as it progresses toward the right. These various anions, which are desirable for use in the facilitated transport membrane according to the present invention due to low lattice energy, have been widely utilized in electrochemical devices such as batteries or electrochemical capacitors, etc. Such anions may include SCN⁻, ClO₄⁻ , CF₃SO₃⁻, BF4⁻, AsF₆⁻, PF₆⁻, SbF₆⁻, AlCl₄⁻, N(SO₂CF₃)₂⁻, C(SO₂CF₃)₃⁻, etc., but various anions in addition to those illustrated herein may be used in the present invention. Anions coinciding with the object of the present invention are not limited to those described herein.

Further, monosalts as well as complex salts of transition metals, such as (M₁)ₓ(M₂) _{x'}Y₂, (M₁)ₓ(X₁) _{y}(M₂) _{x'}(X₂) _{y'} (wherein M₁ and M₂ represent a cation; X, X₁ and X₂ represent an anion; and x, x', y and y' represent atomic value) or organic salt-transition metal salts, or physical mixtures of at least one salt may be used in the facilitated transport separation of the present invention.

Examples of the complex salts of transition metals may include RbAg₄I₅, Ag₂HgI₄, RbAg₄I₄CN, AgHgSI, AgHgTeI, Ag₃SI, Ag₆I₄WO₄, Ag₇I₄AsO₄, Ag₇I₄PO₄, Ag₁₉I₁₅P₂O₇, Rb₄Cu₁₆I₇Cl₁₃, Rb₃Cu₇Cl₁₀, AgI-(tetraalkyl ammonium iodide), AgI-(CH₃)₃SI, C₆H₁₂N₄·CH₃I-CuI, C₆H₁₂N₄ · 4CH₃Br-CuBr, C₆H₁₂N₄· 4C₂H₅Br-CuBr, C₆H₁₂N₄· 4HCl-CuCl, C₆H₁₂N₂· 2CH₃I-CuI, C₆H₁₂N₂ · 2CH₃Br-CuBr, C₆H₁₂N₂ ·2CH₃Cl-CuCl, C₅H₁₁NCH₃I-CuI, C₅H₁₁NCH₃Br-CuBr, C₄H₉ON· CH₃I-CuI, etc. However, numerous combinations similar to these complex salts or mixtures of salts can be made within the spirit of the present invention. As such, the present invention is not limited to those illustrated above.

The polymer used in the present invention must contain double carbon bonds, as described above, so that it can form a complex with transition metal salts and allow the reversible interaction of transition metal ions and alkenes. That is, the polymer used in the solid electrolyte of the facilitated transport membrane according to the present invention must contain double carbon bonds in order to easily form a complex with transition metal salts. The representative examples of the polymer may include polyhexamethylene vinylene (-(CH₂)₆CH=CH-), polystyrene (-CH₂CH(C₆H₅)-), polytrimethylsilylpropyne (-CH₃C=CSi(CH₃)₃-), polybutadiene (-CH₂CH=CHCH₂-), polyisoprene (-CH₂CH=CCH₃CH₂-), polynorbornene (-C₅H₈CH=CH-), polypynene (-(CH₃)₂C(C₆H₈)CH₂-), etc.

Any polymer that does not depart from the object of the present invention and is selected from these polymers, homopolymers or copolymers thereof, derivatives having the polymers as a backbone or a branch, or physical mixtures of the polymers, etc., may be used in the facilitated transport membrane of the present invention. Also, various polymers in addition to the polymers illustrated above may be used in the membrane. Thus, polymers coinciding with the object of the present invention are not limited to those described herein.

The facilitated transport membrane according to the present invention is prepared by applying a polymer electrolyte solution on a porous supported membrane and then drying it. The polymer electrolyte solution that is used in preparing the facilitated transport membrane is prepared by dissolving a transition metal salt and a polymer having double carbon bonds in a liquid solvent to prepare a coating solution. Any liquid solvent that does not impair the supported membrane and can dissolve the transition metal and polymer can be used as a liquid solvent in the process.

The various methods that are well known in the art can be used in applying the electrolyte coating solution on the supported membrane. For example, blade/knife coating, Mayer bar coating, dip coating, air knife coating, etc., can be conveniently used in this regard.

The thickness of the solid electrolyte formed on the supported membrane after drying is preferably as thin as possible in order to enhance permeance. If the dry thickness of the solid electrolyte layer is too thin, however, all pores of a porous support membrane are not blocked or punctures occur in the membrane due to a pressure difference in operation, resulting in selectivity deterioration. Therefore, the dry thickness of said layer is preferably in the range of from 0.05 *µ*m to 10 *µ*m, more preferably in the range of from 0.1 µm to 3 µm.

Another feature of the facilitated transport membrane is high selective permeance for alkenes. The facilitated transport membrane prepared according to the present invention exhibits very high selectivity to alkene hydrocarbons, which is superior to prior selectivity to alkene hydrocarbons, and sustains its activity even in a completely dry state because the solid electrolyte consists of a metal salt and a non-volatile polymer. Further, the facilitated transport membrane is suitable for the practical separation process of alkane/alkene since long-term operation stability is high due to the absence of components that can be volatilized during operation.

The examples below illustrate the present invention in detail, but the invention is not limited to the scope thereof.

### EXAMPLE 1

0.1g of polyhexamethylene vinylene (PHMV, M_{w} = 100,000, T_{g} = -65°C, Tₘ = 58°C, Aldrich Co.) was dissolved in 0.9g of tetrahydrofuran (THF) to obtain a uniform and clear polymer solution (polymer concentration =10 wt%).

Then, 0.089g of silver tetrafluoroborate (AgBF₄, 98%, Aldrich Co.) was added to the solution to obtain [C=C]:[Ag] = 2:1 in mole ratio. The resulting solution was coated on a polyester porous membrane (track-etched membrane, 0.1 *µ*m polyester, Whatman) by using a Mayer bar. The thickness of substantial separation layer determined by a high resolution electron microscope (SEM) was about 2.8 *µ*m. The separation membrane thus prepared was completely dried in a dry oven for 2 hrs and a vacuum oven for 48 hrs at room temperature.

The permeance to pure gas of the prepared membrane was measured at room temperature under conditions in which the pressure of the top portion was 60 psig and the pressure of the permeation portion was 0 psig. In addition, gas permeance was measured with a soap-bubble flow meter. The results expressed in GPU [10⁻⁶ cm³(STP)/cm² ·cmHg· sec] are shown in Table 3 below. Propane permeance was below the measurement limit and, thus, regarded to be below 0.1 GPU.

**Table 3**

| Separation membrane | Permeance to pure gas (GPU) | | | | Selectivity to pure gas | |
|---|---|---|---|---|---|---|
| | Propylene | Ethylene | Propane | Ethane | Propylene/ Propane | Ethylene/ Ethane |
| 2:1 PHMV: AgBF₄ | 33.6 | 6.9 | <0.1 | <0.1 | >336 | >69 |

### EXAMPLE 2

A PHMV/AgClO₄ separation membrane was prepared by using the method described in Example 1. 0.1 g of polyhexamethylene vinylene (PHMV) was dissolved in 0.9g of tetrahydrofuran (THF) to obtain a uniform and clear polymer solution (polymer concentration = 10 wt%).

Then, 0.094g of silver perchlorate (AgClO₄, 99.9%, Aldrich Co.) was added to the solution to obtain [C=C]:[Ag] = 2:1 in mole ratio. The resulting solution was coated on a polyester porous membrane by using a Mayer bar. The separation membrane thus prepared was completely dried in a dry oven for 2 hrs and a vacuum oven for 48 hrs at room temperature.

The permeance to pure gas of the resulting membrane was measured at room temperature under conditions in which the pressure of the top portion was 60 psig and the pressure of the permeation portion was 0 psig. In addition, gas permeance was measured with a soap-bubble flow meter. The results expressed in GPU [10⁻⁶ cm³(STP)/cm²· cmHg·sec] are shown in Table 4 below. Propane permeance was below the measurement limit and, thus, regarded to be below 0.1 GPU.

**Table 4**

| Separation membrane | Permeance to pure gas (GPU) | | | | Selectivity to pure gas | |
|---|---|---|---|---|---|---|
| | Propylene | Ethylene | Propane | Ethane | Propylene/ Propane | Ethylene/ Ethane |
| 2:1 PHMV: AgClO₄ | 16.8 | 3.5 | <0.1 | <0.1 | >168 | >35 |

### EXAMPLE 3

A PHNW/AgCF₃SO₃ separation membrane was prepared by using the method described in Example 1. 0.1 g of polyhexamethylene vinylene (PHMV) was dissolved in 0.9g of tetrahydrofuran (THF) to obtain a uniform and clear polymer solution (polymer concentration = 10 wt%).

Then, 0.117g of silver trifluoromethane sulfonate (AgCF₃SO₃ or AgTf, 99+%, Aldrich Co.) was added to the solution to obtain [C=C]:[Ag] = 2:1 in mole ratio. The resulting solution was coated on a polyester porous membrane by using a Mayer bar. The separation membrane thus prepared was completely dried in a dry oven for 2 hrs and a vacuum oven for 48 hrs at room temperature.

The permeance to pure gas of the resulting membrane was measured at room temperature under conditions wherein the pressure of the top portion was 60 psig and the pressure of the permeation portion was 0 psig. In addition, gas permeance was measured with a soap-bubble flow meter. The results expressed in GPU [10⁻⁶ cm³(STP)/cm² · cmHg · sec] are shown in Table 5 below. Propane permeance was below the measurement limit and, thus, regarded to be below 0.1 GPU.

**Table 5**

| Separation membrane | Permeance to pure gas (GPU) | | | | Selectivity to pure gas | |
|---|---|---|---|---|---|---|
| | Propylene | Ethylene | Propane | Ethane | Propylene/ Propane | Ethylene/ Ethane |
| 2:1 PHMV: AgTf | 12.8 | 1.0 | <0.1 | <0.1 | >128 | >10 |

### EXAMPLE 4

A PHMV/AgSbF₆ separation membrane was prepared by using the method described in Example 1. 0.1 g of polyhexamethylene vinylene (PHMV) was dissolved in 0.9g of tetrahydrofuran (THF) to obtain a uniform and clear polymer solution (polymer concentration = 10 wt%).

Then, 0.156g of silver hexafluoroantimoninate (AgSbF₆, 98%, Aldrich Co.) was added to the solution to obtain [C=C]:[Ag] = 2:1 in mole ratio. The prepared solution was coated on a polyester porous membrane by using a Mayer bar. The separation membrane thus prepared was completely dried in a dry oven for 2 hrs and a vacuum oven for 48 hrs at room temperature.

The permeance to pure gas of the resulting membrane was measured at room temperature under conditions wherein the pressure of the top portion was 60 psig and the pressure of the permeation portion was 0 psig. In addition, gas permeance was measured with a soap-bubble flow meter. The results expressed in GPU [10⁻⁶ cm³(STP)/cm²·cmHg·sec] are shown in Table 6 below. The propane permeance was below the measurement limit and, thus, regarded to be below 0.1 GPU.

**Table 6**

| Separation membrane | Permeance to pure gas (GPU) | | | | Selectivity to pure gas | |
|---|---|---|---|---|---|---|
| | Propylene | Ethylene | Propane | Ethane | Propylene/ | Ethylene/ Ethane |
| 2:1 PHMV: AgSbF₆ | 14.7 | 0.36 | <0.1 | <0.1 | >147 | >3.6 |

### EXAMPLE 5

The separation membrane prepared in Example 1 was examined on the pressure-dependency of permeance to pure gas at room temperature. The gas permeance was measured with a soap-bubble flow meter. The results expressed in GPU [10⁻⁶ cm³(STP)/cm²· cmHg· sec] are shown in Table 7 below. The propane permeance was below the measurement limit and, thus, regarded to be below 0.1 GPU.

As seen in Table 7, the permeance of alkene hydrocarbon was much higher than that of alkene hydrocarbon, i.e., propane. Also, permeance increased with increasing pressure. In particular, gas permeance decreased in the order of 1,3-butadiene, propylene and ethylene.

**Table 7**

| Pressure (psig) | Permeance to pure gas (GPU) | | | |
|---|---|---|---|---|
| | 1,3-Butadiene | Propylene | Ethylene | Propane |
| 10 | 31.9 | 1.5 | <0.1 | <0.1 |
| 20 | 47.8 | 7.9 | 0.3 | <0.1 |
| 30 | - | 19.8 | 4.5 | <0.1 |
| 40 | - | 23.5 | 6.2 | <0.1 |
| 50 | - | 30.1 | 6.7 | <0.1 |
| 60 | - | 33.6 | 6.9 | <0.1 |
| 70 | - | 41.9 | 7.2 | <0.1 |

### EXAMPLE 6

The separation membrane prepared in Example 1 was examined the silver ion concentration-dependency of permeance to pure gas at room temperature. 0.1 g of polyhexamethylene vinylene (PHMV) was dissolved in 0.9 g of tetrahydrofuran (THF) to obtain a uniform and clear polymer solution. The solution was divided into five (5) solutions. 0.03 g, 0.044 g, 0.059 g or 0.089 g of silver tetrafluoroborate (AgBF₄) was added to four (4) of the solutions to obtain the solutions of [C=C]:[Ag] = 6:1, 4:1, 3:1 or 2:1 in mole ratio and a solution containing no silver tetrafluoroborate.

Each of the prepared solutions was coated on a polyester porous membrane by using a Mayer bar. Gas permeance of the membranes was measured at room temperature under conditions wherein the pressure of the top portion was 20 psig and the pressure of the permeation portion was 0 psig. The results are shown in Table 8 below.

As seen in Table 8, facilitated transport phenomena were exhibited at [C=C]:[Ag] = 4:1 in mole ratio, and separation performance increased along with the increase in silver content and decreased at 1:1 in mole ratio.

**Table 8**

| Composition [C=C]:[Ag] | Permeance to pure gas (GPU) | | | | Selectivity |
|---|---|---|---|---|---|
| | 1,3-Butadiene | Propylene | Ethylene | Propane | Propylene/Propane |
| No Ag | 63.7 | 54.1 | 47.3 | 45.5 | 1.2 |
| 6:1 | 52 | 41.0 | 43.3 | 33.6 | 1.2 |
| 4:1 | 11.2 | 10.7 | 6.1 | 5.3 | 2.0 |
| 3:1 | 34.4 | 5.9 | 0.1 | <0.1 | 59 |
| 2:1 | 47.8 | 7.9 | 0.5 | <0.1 | 78.6 |
| 1:1 | 52.3 | 9.8 | 5.7 | 3.1 | 3.2 |

### EXAMPLE 7

The separation membrane prepared in Example 1 was examined on a permeance and selectivity to a gas mixture at room temperature. The separation performance was tested using a propylene/propane mixture (50:50 vol%). The permeance of a permeated gas was determined with a soap-bubble flow meter, and the composition ratio was determined with gas chromatography. The results are shown in Table 9 below.

As seen in Table 9, permeance to a gas mixture increased with increasing pressure, while selectivity was not greatly affected by pressure.

**Table 9**

| Pressure (psig) | Permeance to a gas mixture (GPU) | Selectivity to a gas mixture (propylene/propane) |
|---|---|---|
| 10 | 0.3 | 18.5 |
| 20 | 1.2 | 17.3 |
| 30 | 4.6 | 16.8 |
| 40 | 7.0 | 18.1 |
| 50 | 10.2 | 17.4 |

### EXAMPLE 8

The separation membrane prepared in Example 1 was examined on a long-term operation performance at room temperature. The separation performance was tested using a propylene/propane mixture (50:50 vol%) under conditions wherein the pressure of the top portion was 60 psig and the pressure of permeation the portion was 0 psig.

The permeance of a permeated gas was determined with a soap-bubble flow meter, and the composition ratio was determined with gas chromatography to evaluate the long-term operation performance. Also, a poly(2-ethyl-2-oxazole) (POZ)/AgBF₄ separation membrane having a functional group including oxygen, which is not according to the present invention, was examined on a long-term operation performance as described above. The results are shown in Table 10 below.

As seen in Table 10, the permeance and selectivity of the POZ/AgBF₄ separation membrane continuously decreased with time, while the performance of the PHMV/AgBF₄ separation membrane barely decreased and was maintained during a long-term operation of about 150 hrs.

**Table 10**

| Time (hr) | PHMV/AgBF₄ | | POZ/AgBF₄ | |
|---|---|---|---|---|
| | Permeance to a gas mixture (GPU) | Selectivity to a gas mixture (propylene/propane) | Permeance to a gas mixture (GPU) | Selectivity to gas mixture (propylene/propane) |
| 2 | 10.3 | 17.3 | 16 | 52 |
| 6 | 8.2 | 18.6 | 15 | 52 |
| 12 | 8.7 | 17.5 | 12 | 51 |
| 24 | 9.4 | 16.1 | 13 | 48 |
| 48 | 8.8 | 16.4 | 12 | 42 |
| 72 | 8.7 | 16.4 | 7 | 37 |
| 96 | 9.9 | 15.7 | 5 | 34 |
| 120 | 10.3 | 15.2 | 4 | 31 |
| 144 | 10.1 | 15.9 | 3 | 29 |

### EXAMPLE 9

0.1 g of polystyrene (M_{w} = 280,000, T_{g} = 100°C, Aldrich Co.) was dissolved in 0.9g of tetrahydrofuran (THF) to obtain a uniform and clear polymer solution (polymer concentration = 10 wt%).

0.094g of silver tetrafluoroborate (AgBF₄) was added to the solution to obtain [C=C]:[Ag] = 2:1 in mole ratio. The prepared solution was coated on a polyester porous membrane by using a Mayer bar. The separation membrane thus prepared was completely dried in a dry oven for 2 hrs and a vacuum oven for 48 hrs at room temperature.

The permeance and selectivity to pure gas and gas mixture of the membrane were measured at room temperature under conditions wherein the pressure of the top portion was 40 psig and the pressure of the permeation portion was 0 psig. Gas permeance was measured with a soap-bubble flow meter. The results expressed in GPU [10⁻⁶ cm³(STP)/cm²· cmHg· sec] are shown in Table 11 below. Propane permeance was below the measurement limit and, thus, regarded to be below 0.1 GPU.

**Table 11**

| Separation membrane | Permeance to pure gas (GPU) | | | | Permeance and selectivity to a gas mixture (propylene/propane) | |
|---|---|---|---|---|---|---|
| | Propylene | Propane | Ethylene | Ethane | Permeance | Selectivity |
| Pure PS | <0.1 | <0.1 | <0.1 | <0.1 | <0.1 | 2.8 |
| 1:1 PS/AgBF₄ | 9.9 | <0.1 | 4.1 | <0.1 | 4.6 | 63.0 |

The facilitated transport membrane prepared according to the present invention exhibits very high selectivity to alkene hydrocarbons, which is superior to the prior selectivity to alkene hydrocarbons. Furthermore, no problems, e.g., reduction of a transition metal ion to a transition metal, arose in using a polymer matrix having a functional group containing oxygen and/or nitrogen because the solid polymer matrix of the membrane contains double carbon bonds as a functional group.

While the present invention has been shown and described with respect to particular examples, it will be apparent to those skilled in the art that many changes and modifications can be made without departing from the scope of the invention as defined in the appended claims.

## Claims

1. Use of a facilitated transport membrane comprising a porous support membrane and a solid polymer electrolyte layer consisting of a transition metal salt and a polymer having double carbon bonds,
for separating alkene hydrocarbons from hydrocarbon mixtures,
**characterized in that**
said separating is under dry operating conditions without feed of liquid solvents, and **in that**
said polymer is selected from the group consisting of polytrimethylsilylpropyne, polystyrene, poly(tert-butyl) propyne, polyisopropylpropyne, polybutadiene, polyisoprene, polynorbornene, polyhexamethylene vinylene, polypynene and mixtures thereof.

2. The use according to claim 1, wherein a cation of the transition metal salt has the electronegativity of 1.8∼2.3.

3. The use according to claim 2, wherein the transition metal is one selected from the group consisting of Mn, Fe, Co, Ni, Cu, Mo, Tc, Ru, Rh, Pd, Ag, Re, Os, Ir, Pt and complexes thereof.

4. The use according to claim 1, wherein the transition metal salt has a lattice energy of 2500 kJ/mol or less.

5. The use according to claim 4, wherein an anion of the transition metal salt is one selected from the group consisting of F⁻, Cl⁻, Br⁻, I⁻, CN⁻, NO₃⁻, SCN⁻, ClO₄⁻, CF₃SO₃⁻, BF₄⁻, AsF₆⁻, PF₆⁻, SbF₆⁻, AlCl₄⁻, N(SO₂CF₃)₂⁻ and C(SO₂CF₃)₃⁻.

6. The use according to claim 1, wherein the transition metal salt includes a complex salt of the transition metal or a mixture of the salts of the transition metal.

7. The use according to claim 1, wherein the porous support membrane is a porous polymer membrane or ceramic membrane.

8. The use according to claim 1, wherein the hydrocarbon mixtures to be separated contain at least one alkene hydrocarbon and at least one alkane hydrocarbon or an inert gas.

9. The use according to claim 8, wherein the alkene hydrocarbon is one selected from the group consisting of ethylene, propylene, butylene, 1,3-butadiene, isobutylene and mixtures thereof, the alkane hydrocarbon is one selected from the group consisting of methane, ethane, propane, butane, isobutane and mixtures thereof, and the inert gas is one selected from the group consisting of oxygen, nitrogen, carbon dioxide, carbon monoxide and mixtures thereof.

## Patentansprüche

1. Verwendung einer Membran zum erleichterten Transport, umfassend eine poröse Stützmembran und eine feste polymere Elektrolytschicht, die aus einem Übergangsmetallsalz und einem Polymer mit doppelten Kohlenstoffbindungen besteht,
zum Trennen von Alkenkohlenwasserstoffen von Kohlenwasserstoffgemischen,
**dadurch gekennzeichnet, dass**
das Trennen unter trockenen Betriebsbedingungen erfolgt, ohne Zufuhr flüssiger Lösemittel, und dass
das Polymer ausgewählt ist aus der Gruppe bestehend aus Polytrimethylsilylpropin, Polystyrol, Poly(tert-butyl)-propin, Polyisopropylpropin, Polybutadien, Polyisopren, Polynorbornen, Polyhexamethylenvinylen, Polypinen und Gemischen davon.

2. Verwendung gemäß Anspruch 1, wobei ein Kation des Übergangsmetallsalzes die Elektronegativität von 1,8 - 2,3 aufweist.

3. Verwendung gemäß Anspruch 2, wobei das Übergangsmetall ausgewählt ist aus der Gruppe bestehend aus Mn, Fe, Co, Ni, Cu, Mo, Tc, Ru, Rh, Pd, Ag, Re, Os, Ir, Pt und Komplexen davon.

4. Verwendung gemäß Anspruch 1, wobei das Übergangsmetallsalz eine Gitterenergie von 2500 kJ/mol oder weniger hat.

5. Verwendung gemäß Anspruch 4, wobei ein Anion des Übergangsmetallsalzes ausgewählt ist aus der Gruppe bestehend aus F⁻, Cl⁻, Br⁻, I⁻, CN⁻, NO₃⁻, SCN⁻, ClO₄⁻, CF₃SO₃⁻, BF₄⁻, AsF₆⁻, PF₆⁻, SbF₆⁻, AlCl₄⁻, N(SO₂CF₃)₂⁻ und C(SO₂CF₃)₃⁻.

6. Verwendung gemäß Anspruch 1, wobei das Übergangsmetallsalz ein komplexes Salz des Übergangsmetalls oder ein Gemisch der Salze des Übergangsmetalls enthält.

7. Verwendung gemäß Anspruch 1, wobei die poröse Stützmembran eine poröse Polymermembran oder keramische Membran ist.

8. Verwendung gemäß Anspruch 1, wobei die zu trennenden Kohlenwasserstoffgemische mindestens einen Alkenkohlenwasserstoff und mindestens einen Alkankohlenwasserstoff oder ein inertes Gas enthalten.

9. Verwendung gemäß Anspruch 8, wobei der Alkenkohlenwasserstoff ausgewählt ist aus der Gruppe bestehend aus Ethylen, Propylen, Butylen,
1,3-Butadien, Isobutylen und Gemischen davon, wobei der Alkankohlenwasserstoff ausgewählt ist aus der Gruppe bestehend aus Methan, Ethan, Propan, Butan, Isobutan und Gemischen davon, und das inerte Gas ausgewählt ist aus der Gruppe bestehend aus Sauerstoff, Stickstoff, Kohlendioxid, Kohlenmonoxid und Gemischen davon.

## Revendications

1. Utilisation d'une membrane pour transport facilité comprenant une membrane support poreuse et une couche d'électrolyte polymère solide consistant en un sel d'un métal de transition et d'un polymère ayant des double-liaisons carbonées,
pour séparer des hydrocarbures alcènes de mélanges d'hydrocarbures,
**caractérisé en ce que**
ladite séparation se fait dans des conditions d'opération à sec sans fourniture de solvants liquides, et **en ce que**
ledit polymère est sélectionné dans le groupe consistant en le polytriméthylsilylpropyne, le polystyrène, le poly(tert-butyl)propyne, le polyisopropylpropyne, le polybutadiène, le polyisoprène, le polynorbornène, le polyhexaméthylène vinylène, le polypynène et leurs mélanges.

2. Utilisation selon la revendication 1, dans laquelle un cation du sel de métal de transition a une électronégativité de 1,8∼2,3.

3. Utilisation selon la revendication 2, dans laquelle le métal de transition est sélectionné dans le groupe consistant en Mn, Fe, Co, Ni, Cu, Mo, Tc, Ru, Rh, Pd, Ag, Re, Os, Ir, Pt et leurs complexes.

4. Utilisation selon la revendication 1, dans laquelle le sel de métal de transition a une énergie de réseau de 2500 kJ/mol ou moins.

5. Utilisation selon la revendication 4, dans laquelle un anion du sel de métal de transition est sélectionné dans le groupe consistant en F⁻, Cl⁻, Br⁻, I⁻, CN⁻, NO₃⁻, SCN⁻, ClO₄⁻, CF₃SO₃⁻, BF₄⁻, AsF₆⁻, PF₆⁻, SbF₆⁻, AlCl₄⁻, N(SO₂CF₃)₂⁻ et C(SO₂CF₃)₃⁻.

6. Utilisation selon la revendication 1, dans laquelle le sel de métal de transition inclut un sel complexe du métal de transition ou un mélange de sels du métal de transition.

7. Utilisation selon la revendication 1, dans laquelle la membrane de support poreuse est une membrane polymère poreuse ou une membrane céramique.

8. Utilisation selon la revendication 1, dans laquelle les mélanges d'hydrocarbures à séparer contiennent au moins un hydrocarbure alcène et au moins un hydrocarbure alcane ou un gaz inerte.

9. Utilisation selon la revendication 8, dans laquelle l'hydrocarbure alcène est sélectionné dans le groupe consistant en l'éthylène, le propylène, le butylène, le 1,3-butadiène, l'isobutylène et leurs mélanges, l'hydrocarbure alcane est sélectionné dans le groupe consistant en le méthane, l'éthane, le propane, le butane, l'isobutane et leurs mélanges, et le gaz inerte est sélectionné dans le groupe consistant en l'oxygène, l'azote, le dioxyde de carbone, le monoxyde de carbone et leurs mélanges.
